# EUROPEAN PATENT APPLICATION

(11) **EP 3 973 909 A1**
(43) Date of publication of application: **30.03.2022**
(21) Application number: 21199500.6
(22) Date of filing: 28.09.2021
(51) Int. Cl.: A61B 34/20, A61M 25/01, A61B 17/00

(54) **CIRCULAR NAVIGATION CATHETER WITH SURFACE MOUNTED INDUCTIVE NAVIGATION SENSORS**

(30) Priority: 29.09.2020 US 202063084674 P; 16.09.2021 US 202117477272
(71) Applicant: Biosense Webster (Israel) Ltd, Yokneam, 2066717 (IL)
(72) Inventor: HIGHSMITH, Debby, Irvine, 92618 (US); BAR-TAL, Meir, 2066717 Yokneam (IL)
(74) Representative: Carpmaels & Ransford LLP

(57) **Abstract**

A catheter is presented herein which includes inductive coils which conform to the curved surface of a tubular catheter body and collectively can function as a three axis sensor during an intravascular and/or intracardiac treatment. The inductive coils can be fabricated on a flexible circuit substrate and affixed to the tubular catheter body. The catheter can include a distal portion that can be moved into a circular shape ("lasso") when within vasculature or the heart. The inductive coils can be positioned around the circular shape such that a position and orientation of the distal portion can be determined in three dimensions when the distal portion is within a known fluctuating magnetic field.

## Description

### FIELD

The present application relates generally to electronic circuitry, and specifically to electronic circuitry of magnetic field sensors. The present application further relates to catheters including magnetic field sensors.

### BACKGROUND

A magnetic field can be sensed by positioning a conductive coil in the magnetic field and observing electrical current and/or voltage induced in the coil by a change in the magnetic field that is aligned with an axis of the conductive coil. Because electrical current is induced in the conductive coil, such a coil is also referred to as an inductive coil. Relative position of a sensor including one or more inductive coils can be determined in relation to a known magnetic field source by monitoring the induced electric current and/or voltage.

Navigation sensors which include three coil arrangements aligned along three orthogonal axes to determine position and orientation of the navigation sensor in three dimensions within a known induced magnetic field are disclosed for instance in U.S. Patent Numbers 10,330,742 and 10,677,857, each of which are hereby incorporated herein by reference in its entirety into this application as if set forth in full herein and which are attached in the Appendix to priority application U.S. 63/084,674.

Integrating a navigation sensor into a catheter suitable for intravascular and/or intracardiac treatments can involve crafting the sensor by hand, which can result in significant manufacturing time and labor costs. Navigation sensor components can account for a significant percentage of catheter material costs.

### SUMMARY

A catheter is presented herein which includes inductive coils which conform to the curved surface of a tubular catheter body and collectively can function as a three axis sensor during an intravascular treatment. The inductive coils can be fabricated on a flexible circuit substrate and affixed to the tubular catheter body. The catheter can include a distal portion that can be moved into a circular shape ("lasso") when within vasculature or the heart. The inductive coils can be positioned around the circular shape such that a position and orientation of the distal portion can be determined in three dimensions when the distal portion is within a known magnetic field.

An example catheter can have a tubular body and a circuit. The tubular body can have a proximal shaft and a distal portion. The tubular body can have a delivery configuration in which the distal portion and proximal shaft are aligned along a longitudinal axis. The proximal shaft can be manipulated to deliver the distal portion through vasculature. The distal portion can have a cylindrical surface with a curvature that curves around the longitudinal axis when the tubular body is in the delivery configuration. The circuit can include a first inductive sensor, a second inductive sensor, and a third inductive sensor. The sensors, collectively, can be used to determine position and orientation of the distal portion in three dimensions when the distal portion is within a known magnetic field. In other words, the sensors, collectively, can function as a three-axis sensor. The circuit can be affixed to the cylindrical surface such that the first inductive sensor, the second inductive sensor, and the third inductive sensor each conform to the curvature of the cylindrical surface.

The tubular body can have a deployed configuration in which the distal portion has a generally circular shape. The circular shape can be generally orthogonal to the longitudinal axis defined by the proximal shaft of the tubular body. The tubular body can be movable from the delivery configuration to the deployed configuration via manipulation of the proximal shaft. When the distal portion is in the generally circular shape, the first inductive sensor, second inductive sensor, and third inductive sensor can be spaced approximately equidistant from each other around the generally circular shape. The generally circular shape can have a circumference measuring approximately 50 millimeters.

The catheter can further include a support member extending through a tubular lumen within the distal portion of the tubular body. The tubular body can include a flexible polymeric material. The support member can include a memory shape material. The memory shape material can have a predetermined shape that is shaped approximately the same as the generally circular shape. The catheter can further include a contraction wire extending through the tubular lumen of the tubular body within the distal portion. The contraction wire can be moved to cause the distal portion to become shaped into the generally circular shape.

The first inductive sensor, the second inductive sensor, and the third inductive sensor can each lack any inductive coil circumscribing the cylindrical surface.

The first inductive sensor can include first inductive coils spiraling substantially parallel to the cylindrical surface such that the first inductive coils conform to the cylindrical surface. The second inductive sensor can include second inductive coils spiraling substantially parallel to the cylindrical surface such that the second inductive coils conform to the cylindrical surface. The third inductive sensor can include third inductive coils spiraling substantially parallel to the cylindrical surface such that the third inductive coils conform to the cylindrical surface.

The first inductive coils can include a first coil, a second coil, a third coil, and a fourth coil arranged in a particular arrangement. The second and/or third inductive sensors can respectively include four coils arranged in a similar manner. The first, second, third, and fourth coils can be arranged as follows. The first coil can be positioned on a first side of the cylindrical surface and include a central termination. The second coil can be positioned on a second side of the cylindrical surface, about 180° around the cylindrical surface from the first side and also include a central termination. The first coil can spiral oppositely from the second coil. The third coil can be positioned on the first side of the cylindrical surface. The first coil and third coil can be positioned such that a majority of the first coil overlaps a majority of the third coil. The third coil can include a central termination in immediate electrical contact with the central terminal of the first coil. The third coil can spiral oppositely from the first coil. The fourth coil can be positioned on the second side of the cylindrical surface. The second coil and the fourth coil can be positioned such that a majority of the second coil overlaps a majority of the fourth coil. The fourth coil can include a central termination in immediate electrical contact with the central terminal of the second coil. The fourth coil can spiral oppositely from the second coil. The third coil can spiral oppositely from the fourth coil. The third coil and the fourth coil can be confined between two electrically insulative, substantially parallel, arcuate surfaces.

The catheter can further include conductive traces to the coils. A first conductive trace can be in immediate electrical contact with the first coil and extend from the first coil to the proximal shaft. A second conductive trace can be in immediate electrical contact with the second coil and extend from the second coil to the proximal shaft. A third conductive trace can be in immediate electrical contact with the third coil and extend from the third coil to the proximal shaft. The first and third conductive traces can be positioned such that a majority of the first conductive trace overlaps a majority of the third conductive trace. A fourth conductive trace can be in immediate electrical contact with the fourth coil and can extend from the fourth coil to the proximal shaft. The second and fourth conductive traces can be positioned such that a majority of the second conductive trace overlaps a majority of the fourth conductive trace. The fourth conductive trace can be electrically connected to the third conductive trace near the proximal shaft. Similarly, the catheter can include conductive traces to the coils of the second inductive sensor and/or the third inductive sensor.

The circuit can include an insulative substrate, a lower layer, an insulating mid layer, and an upper layer. The insulative substrate can be affixed to the cylindrical surface. The lower layer can be above the insulative substrate and can include the third coil, third conductive trace, fourth coil, and fourth conductive trace. The insulating mid layer can be above the lower layer and can include vias therethrough. The vias can facilitate immediate electrical contact between the central termination of the first coil and the central termination of the third coil and can facilitate immediate electrical contact between the central termination of the second coil and the central termination of the fourth coil. The upper layer above the insulating mid layer can include the first coil, first conductive trace, second coil, and second conductive trace. The circuit can further include an insulative top layer above the upper layer. Similarly, corresponding coils and traces of the second inductive sensor and/or third inductive sensor can be positioned in the lower layer and upper layer of the circuit.

The catheter can further include contact pads and wires connecting to the coils. A first contact pad connected to the first conductive trace can be positioned in the upper layer of the circuit at or near the proximal shaft. A first wire can be soldered to the first contact pad and can extend through the proximal shaft to a proximal end of the tubular body. A second contact pad connected to the second conductive trace can be positioned in the upper layer of the circuit at or near the proximal shaft. A second wire can be soldered to the second contact pad and can extend through the proximal shaft to the proximal end of the tubular body. Similarly, the catheter can include contact pads and wires to the second inductive sensor and/or third inductive sensor.

The inductive coils of the first inductive sensor, the second inductive sensor, and the third inductive sensor can each respectively spiral around a respective coil axis such that each respective coil axis is approximately orthogonal to the cylindrical surface. Each of the inductive coils can have a respective height, measured in a direction of the respective coil axis, and a respective width, measured orthogonal to the respective coil axis. The width can measure at least ten times greater than the height.

An example method for designing, constructing, or assembling a catheter can include one or more of the following steps executed in various orders as understood by a person skilled in the pertinent art according to the teachings herein. The method can include fabricating a multi-layer flexible circuit having a first coil arrangement, a second coil arrangement, and a third coil arrangement. The fabrication can result in the first, second, and third coil arrangements being linearly arranged to define a longitudinal axis of the multi-layer flexible circuit. Each of the first, second, and third coil arrangements can each respectively include four coils. For each of the coil arrangements, the four coils can each include a respective central termination. The four coils can be arranged such that each of the four coils is next to an adjacently stacked coil and an adjacent coplanar coil. The four coils can be arranged such that the central termination of each of the four coils is in immediate electrical contact with its adjacently stacked coil.

The method can include affixing the multi-layer flexible circuit to a cylindrical surface of a tubular catheter body. As a result of affixing, the longitudinal axis of the multi-layer flexible circuit can be aligned lengthwise with the tubular catheter body. The flexible circuit can have arcuate cross sections through each of the first, second, and third coil arrangements, where the cross sections are orthogonal to the longitudinal axis.

The method can include affixing the multi-layer flexible circuit to the cylindrical surface such that each of the four coils is centered about 180° around a circumference of the tubular body from its adjacent coplanar coil.

The method can include forming a distal portion of the tubular catheter body in a substantially circular shape.

The method can include affixing the multi-layer flexible circuit to the cylindrical surface such that each of the first, second, and third coil arrangements are spaced approximately equidistant from each other around the generally circular shape when the distal portion is in the substantially circular shape.

The method can include configuring a proximal shaft of the tubular catheter body such that the distal portion of the tubular catheter is movable from a substantially straight configuration to the substantially circular shape through manipulation of the proximal shaft.

The method can include forming the distal portion of the tubular catheter body in the substantially circular shape such that the substantially circular shape has a circumference measuring approximately 50 millimeters.

The method can include extending a support member through a tubular lumen of the tubular catheter within the distal portion.

The method can include shaping memory shape material of the support member to have a predetermined shape that approximates the generally circular shape.

The method can include extending a contraction wire through the tubular lumen within the distal portion such that the contraction wire is movable to modify a shape of the distal portion to the generally circular shape.

The method can include affixing the multi-layer flexible circuit to the cylindrical surface such that the first coil arrangement, the second coil arrangement, and the third coil arrangement each lack any inductive coil circumscribing the cylindrical surface.

The method can include affixing the multi-layer flexible circuit to the cylindrical surface such that the first inductive coil arrangement has first inductive coils spiraling substantially parallel to the cylindrical surface so that the first inductive coils conform to the cylindrical surface. The method can include affixing the multi-layer flexible circuit to the cylindrical surface such that the second coil arrangement has second inductive coils spiraling substantially parallel to the cylindrical surface so that the second inductive coils conform to the cylindrical surface. The method can include affixing the multi-layer flexible circuit to the cylindrical surface such that the third inductive coil arrangement has third inductive coils spiraling substantially parallel to the cylindrical surface so that the third inductive coils conform to the cylindrical surface.

The method can include affixing the multi-layer flexible circuit to the cylindrical surface such that the first inductive coil arrangement has coils arranged in a particular arrangement. A first coil can be positioned on a first side of the cylindrical surface. A second coil can be positioned on a second side of the cylindrical surface, about 180° around the cylindrical surface from the first side, the first coil and the second coils being adjacent coplanar coils (e.g. coplanar when the circuit was flat prior to affixing to the cylindrical surface). A third coil can be positioned on the first side of the cylindrical surface such that a majority of the first coil overlaps a majority of the third coil so that the first coil and the third coil are adjacently stacked coils joined electrically at their respective central terminations. The third coil can spiral oppositely from the first coil. A fourth coil can be positioned on the second side of the cylindrical surface such that a majority of the second coil overlaps a majority of the fourth coil so that the second coil and the fourth coil are adjacently stacked coils joined electrically at their respective central terminations. The fourth coil can spiral oppositely from the second coil. The third and fourth coils can be adjacent coplanar coils.

The method can include affixing the multi-layer flexible circuit to the cylindrical surface such that the first coil spirals oppositely from the second coil and the third coil spirals oppositely from the fourth coil.

The method can include confining the third coil and the fourth coil between two electrically insulative, substantially parallel, arcuate surfaces.

The method can include fabricating the multi-layer flexible circuit such that a first conductive trace is in immediate electrical contact with the first coil and the first conductive trace extends from the first coil to the proximal shaft. The method can include fabricating the multi-layer flexible circuit such that a second conductive trace is in immediate electrical contact with the second coil and the second conductive trace extends from the second coil to the proximal shaft. The method can include fabricating the multi-layer flexible circuit such that a third conductive trace is in immediate electrical contact with the third coil, the third conductive trace extends from the third coil to the proximal shaft, and a majority of the first conductive trace overlaps a majority of the third conductive trace. The method can include fabricating the multi-layer flexible circuit such that a fourth conductive trace is in immediate electrical contact with the fourth coil, the fourth conductive trace extends from the fourth coil to the proximal shaft, a majority of the second conductive trace overlaps a majority of the fourth conductive trace, and the fourth conductive trace is in electrical contact with the third conductive trace approximate the proximal shaft.

The method can include fabricating the multi-layer flexible circuit such that the multi-layer flexible circuit includes an insulative substrate, a lower layer above the insulative substrate, an insulating mid layer above the lower layer, and an upper layer above the insulating mid layer. The lower layer can include the third coil, third conductive trace, fourth coil, and fourth conductive trace. The insulating mid layer can include vias therethrough which facilitate immediate electrical contact between the central termination of the first coil and the central termination of the third coil and facilitate immediate electrical contact between the central termination of the second coil and the central termination of the fourth coil. The upper layer can include the first coil, first conductive trace, second coil, and second conductive trace.

The method can include affixing the insulative substrate of the multi-layer flexible circuit to the cylindrical surface of the tubular catheter body.

The method can include affixing an insulative top layer above the upper layer of the multi-layer flexible circuit.

The method can include fabricating the multi-layer flexible circuit such that the multi-layer flexible circuit includes a first contact pad and a second contact pad each positioned in the upper layer near the proximal shaft. The method can include soldering a first wire to the first contact pad. The method can include extending the first wire through the tubular catheter body to a proximal end of the tubular catheter body. The method can include soldering a second wire to the second contact pad. The method can include extending the second wire through the tubular catheter body to a proximal end of the tubular catheter body.

An example method for intracardiac diagnostics can include one or more of the following steps executed in various order as understood by a person skilled in the pertinent art according to the teachings herein. The method can include manipulating a proximal shaft of a catheter to position a distal portion of the catheter within a heart. The method can include receiving position signals from inductive coil arrangements affixed to a cylindrical surface of the distal portion. The coil arrangements can be shaped to have an arcuate cross-section as a result of being affixed to the cylindrical surface. The method can include determining position and orientation of the distal portion based at least in part, or solely on the position signals. The method can include determining a three-dimensional position and a three-dimensional orientation of the distal portion based at least in part, or solely on the position signals.

The method can include forming a distal portion of the catheter into a substantially circular shape such that each of the inductive coil arrangements are positioned around a circumference of the circular shape. An example multi-layer flexible circuit can include three coil arrangements each having four coils, where each of the coils has a central termination. The three coil arrangements can be linearly arranged to define a longitudinal axis of the multi-layer flexible circuit. In each coil arrangement, the four coils of that arrangement can be arranged such that each of the four coils is next to an adjacently stacked coil and an adjacent coplanar coil. The central termination of each of the four coils can be in immediate electrical contact with its adjacently stacked coil.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1A illustrates an example catheter in a deployed configuration according to aspects of the present invention;
Figure 1B illustrates the catheter in a delivery configuration according to aspects of the present invention;
Figures 2A and 2B illustrate two example cross-sections of the catheter as indicated in Figures 1A and 1B according to aspects of the present invention;
Figures 3A and 3B, in combination, illustrate an inductive sensor usable with the catheter according to aspects of the present invention;
Figure 4A is an illustration of an example circuit usable with the catheter according to aspects of the present invention;
Figure 4B is an illustration of the cross section of the circuit as indicated in Figure 4A according to aspects of the present invention;
Figures 5A through 5C are illustrations of a first coil arrangement of a first inductive sensor of the circuit illustrated in Figure 4A according to aspects of the present invention;
Figures 6A through 6C are illustrations of a second coil arrangement of a second inductive sensor of the circuit illustrated in Figure 4A according to aspects of the present invention;
Figures 7A through 7C are illustrations of a third coil arrangement of a third inductive sensor of the circuit illustrated in Figure 4A according to aspects of the present invention;
Figures 8A through 8C are illustrations of traces and contact pads of the circuit illustrated in Figure 4A and 4B according to aspects of the present invention;
Figures 9A through 9C are illustrations of another example circuit usable with the catheter according to aspects of the present invention;
Figures 10A and 10B are illustrations of additional example catheters according to aspects of the present invention; and
Figure 11 is an illustration of a treatment incorporating an example catheter according to aspects of the present invention.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

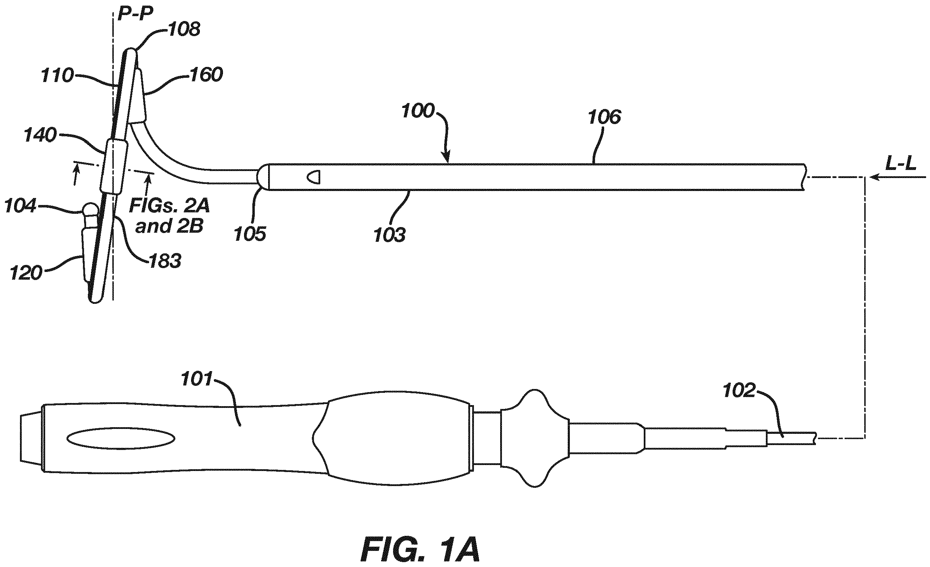

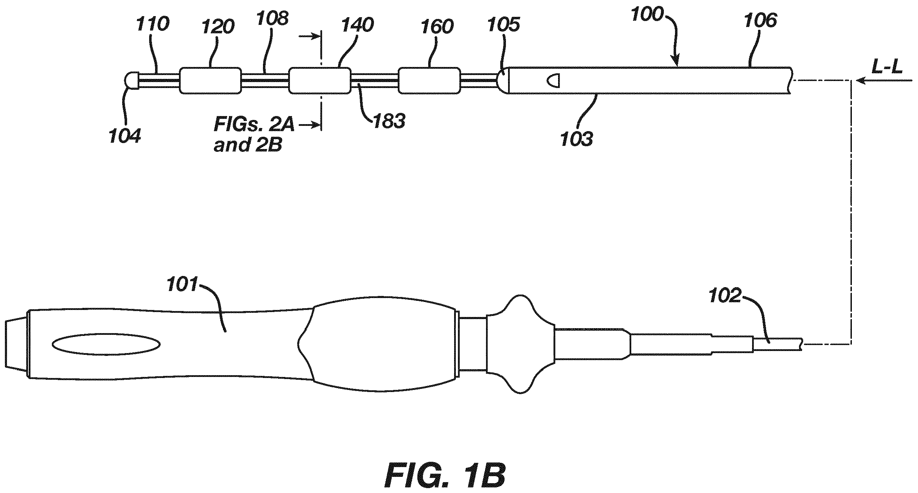

Figures 1A and 1B illustrate an example catheter 100 which includes inductive sensors 120, 140, 160 including inductive coils which conform to the curved surface of a tubular catheter body 103 (specifically to a cylindrical surface 183 of a distal portion 108 of the tubular body 103) and collectively can function as a three axis sensor during an intravascular treatment. The inductive sensors 120, 140, 160 can be fabricated on a flexible circuit 110 and affixed to the tubular catheter body 103. As illustrated in Figure 1A, the distal portion 108 of the tubular body 103 can be moved into a generally circular shape when within vasculature or a heart. The generally circular shape can be slightly helical ("lasso"). The circular shape can curve in the clockwise or counterclockwise direction. The inductive sensors 120, 140, 160 can be positioned around the circular shape such that a position and orientation of the distal portion 108 can be determined in three dimensions when the distal portion 108 is within a known varying magnetic field.

Figure 1B illustrates the tubular body 103 of the catheter 100 in a delivery configuration in which the distal portion 108 and proximal shaft 106 are aligned along a longitudinal axis L-L. The cylindrical surface 183 of the distal portion 108 curves around the longitudinal axis when the tubular body 103 is in the delivery configuration. The circuit 110 can be affixed to the cylindrical surface 183 such that the first inductive sensor 120, the second inductive sensor 140, and the third inductive sensor 160 each conform to the curvature of the cylindrical surface.

The catheter 100 can include a control handle 101 affixed to a proximal end 102 of the tubular body 103 that can be moved to push the tubular body 103 distally through vasculature. In some examples, the control handle 101 can also be used to move the distal portion 108 from the delivery configuration illustrated in Figure 1B to the deployed configuration illustrated in Figure 1A and vice versa similar to a multifunctional catheter handle and corresponding catheter as disclosed in U.S. Patent No. 6,987,995 incorporated herein by reference in its entirety into this application as if set forth in full herein and which is attached in the Appendix to priority application U.S. 63/084,674, or an alternative system capable of causing the distal portion 108 to move to an expanded deployed shape.

As illustrated in Figure 1A, the tubular body 103 can have a deployed configuration in which the distal portion 108 has a generally circular shape. The circular shape can be generally orthogonal to the longitudinal axis L-L defined by the proximal shaft 106 of the tubular body. Alternatively, the circular shape can be aligned to the longitudinal axis L-L or at an oblique angle to the longitudinal axis L-L. As illustrated, the distal portion 108 forms a lasso shape that passes through, and is generally aligned with, a plane P-P that is orthogonal to the longitudinal axis L-L of the shaft 106. Regardless of the angle of the circular shape to the longitudinal axis L-L, when the distal portion 108 is in the generally circular shape, the first inductive sensor 120, second inductive sensor 140, and third inductive sensor 160 can be spaced approximately equidistant from each other around the generally circular shape.

The proximal shaft 106 can have an elongated tubular construction. The proximal shaft 106 can have a single, axial, or central lumen. The proximal shaft 106 can be flexible, i.e., bendable, but substantially non-compressible along its length. The proximal shaft 106 can be of any suitable construction and made of any suitable material. In some examples, the proximal shaft 106 has an outer polymer wall having an interior braided metal mesh. The proximal shaft 106 can have sufficient structural integrity such that when the control handle 101 is rotated, the tubular body 103, including the proximal shaft 106 and distal portion 108, rotate in a corresponding manner. The outer diameter of the proximal shaft 106 is preferably about 8 French or about 7 French.

The useful length of the catheter 100, i.e., that portion that can be inserted into the body, can vary as appropriate based on treatment procedure and anatomy of a patient. For most treatments, the useful length can be about 110 centimeters (cm) to about 120 cm. The length of the distal portion 108 is a relatively small portion of the useful length and preferably is about 3.5 cm to about 10 cm, and more preferably about 5 cm to about 6.5 cm.

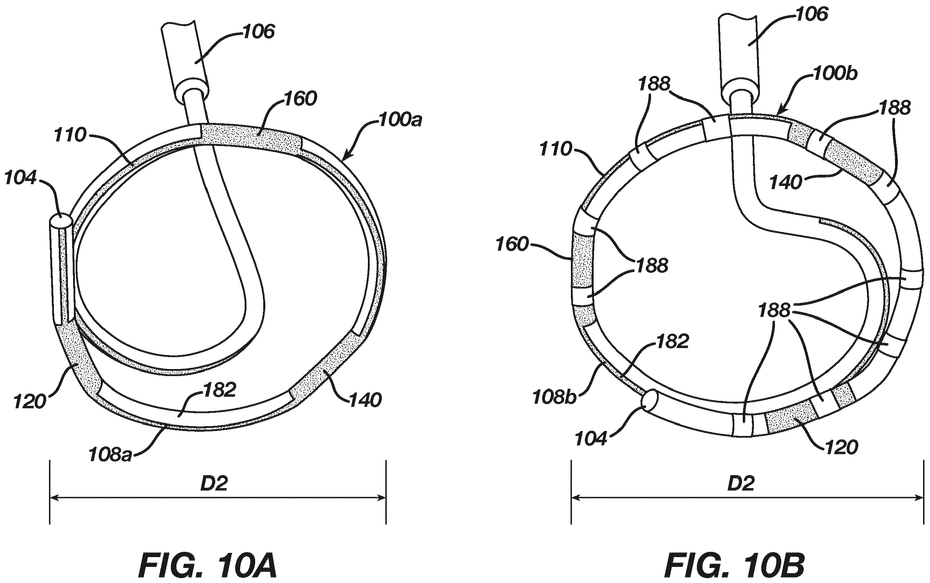

In some examples, the distal portion 108 can have a section aligned with the longitudinal axis L-L, when the distal portion 108 is in the substantially circular shape, measuring about 3 millimeters (mm) to about 12 mm. The distal end 104 of the tubular body 103 may or may not overlap the distal portion 108 when in the circular shape (e.g. comparing Figure 1A with Figures 10A and 10B). The generally circular shape can have a circumference measuring approximately equal to the length of the distal portion 108 or may deviate from the length of the distal portion 108 somewhat. Further, in some examples, the circumference of the circular shape can be modified in patient via manipulation of the control handle 101. The circular shape can have a circumference measuring about 3 cm to about 8 cm, more preferably about 4 cm to about 6 cm, and more preferably about 5 cm.

The proximal shaft 106 and distal portion 108 can be joined with glue or the like. In some examples, the junction 105 can include a spacer similar to as describe in U.S. Patent No. 5,964,757 incorporated herein by reference in its entirety into this application as if set forth in full herein and which is attached in the Appendix to priority application U.S. 63/084,674.

Although not illustrated in Figures 1A and 1B, the distal portion 108 can further include mapping electrodes and associated support structures. For instance, the catheter 100 can include mapping electrodes 188 illustrated in Figure 10B. The mapping electrodes 188 can be positioned and otherwise configured to measure electrical signals from tissue in contact with the distal portion 108.

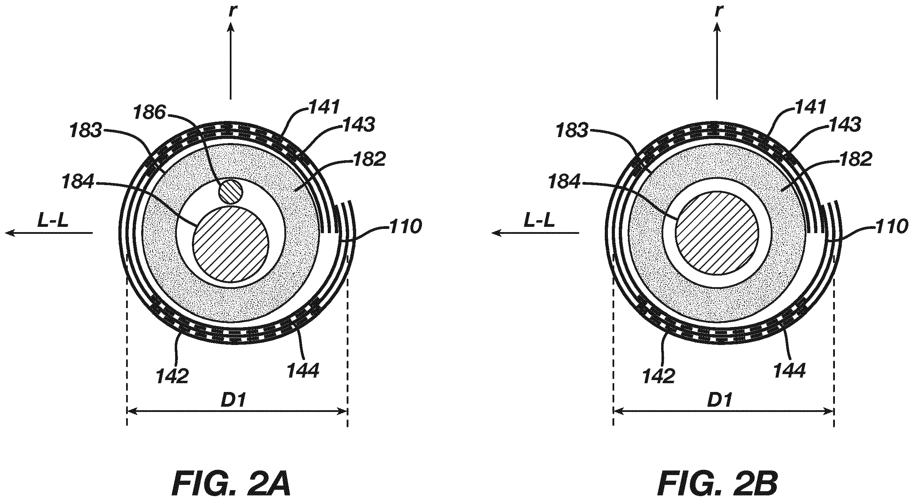

Figures 2A and 2B illustrate example cross-sections of the catheter 100 as indicated in Figures 1A and 1B. The cross-section is indicated as passing through the second inductive sensor 140. The first inductive sensor 120 and third inductive sensor 160 can each have a similar cross section. The catheter 100 can include a support member 184 extending through a tubular lumen within the distal portion 108 of the tubular body 103. Although the distal portion 108 is illustrated having a single lumen, the distal portion 108 can include additional lumens and the number of lumens can change along the length of the distal portion 108. The interior of the distal portion 108 can be configured to accommodate the support member 184, a contraction wire 186 (Figure 2A), electrical conductors, and other desired wires, cables, and/or tubes. Other suitable configurations of the interior of the distal portion 108 are disclosed in U.S. Patent No. 6,987,995 incorporated herein by reference in its entirety into this application as if set forth in full herein and which is attached in the Appendix to priority application U.S. 63/084,674

The support member 184 can include a memory shape material. The memory shape material can have a predetermined shape that is shaped approximately the same as the generally circular shape of the distal portion in the deployed configuration as illustrated in Figure 1A.

Figure 2A illustrates the catheter 100 further including a contraction wire 186 through the lumen. The contraction wire 186 can extend to the distal end 104 of the tubular body 103 or through only a portion of the distal portion 108. The distal portion 108 of the tubular body 103 can include a flexible polymeric tube 182 having sufficient flexibility to move when the contraction wire 186 is pulled and/or when the support member 184 reshapes. When the catheter 100 includes the contraction wire 186, the support member 184 can, but need not, include memory shape material in order to achieve the circular shape illustrated in Figure 1A. The contraction wire 186 can be moved to cause the distal portion 108 to become shaped into the generally circular shape. When the support member 184 includes memory shape material and the catheter 100 includes the contraction wire 186, the contraction wire can function to move the distal portion 108 into a shape which deviates from the predetermined shape of the support member 184.

Figure 2B illustrates the catheter 100 lacking the contraction wire 186 through at least a portion of the distal portion 108 of the tubular body 103 of the catheter 100. The shape of the distal portion 108 can be determined by the shape of the support member 184.

Figures 2A and 2B illustrate a cross section of the circuit 110 through the second inductive sensor 140. Coils 141-144 of the second inductive sensor 140 spiral substantially parallel to the cylindrical surface 183 of the polymer tube 182. The coils 141-144 conform to the cylindrical surface 183 of the polymer tube 182 by virtue of the circuit 110 being wrapped around, and affixed to, the cylindrical surface 183. Similarly, coils 121-124, 161-164 of the first and third inductive sensors 120, 160 can conform to the cylindrical surface 183 of the polymer tube 182 by virtue of the circuit 110 being wrapped around, and affixed to, the cylindrical surface 183. The first inductive sensor 120, the second inductive sensor 140, and the third inductive sensor 160 can each lack any inductive coil circumscribing the cylindrical surface. Each of the coils 121-124, 141, 144, 161-164 can be confined between insulative layers having arcuate cross section.

The coils can spiral around a radial axis (r) that is orthogonal to the cylindrical surface 183. When in the deployed configuration as illustrated in Figure 1A, the coils can be aligned in relation to the longitudinal axis L-L of the shaft 106 as indicated in Figure 2A and 2B. Aligned as such, the radial axis (r) is approximately in the plane P-P of the distal portion 108 as illustrated in Figure 1A.

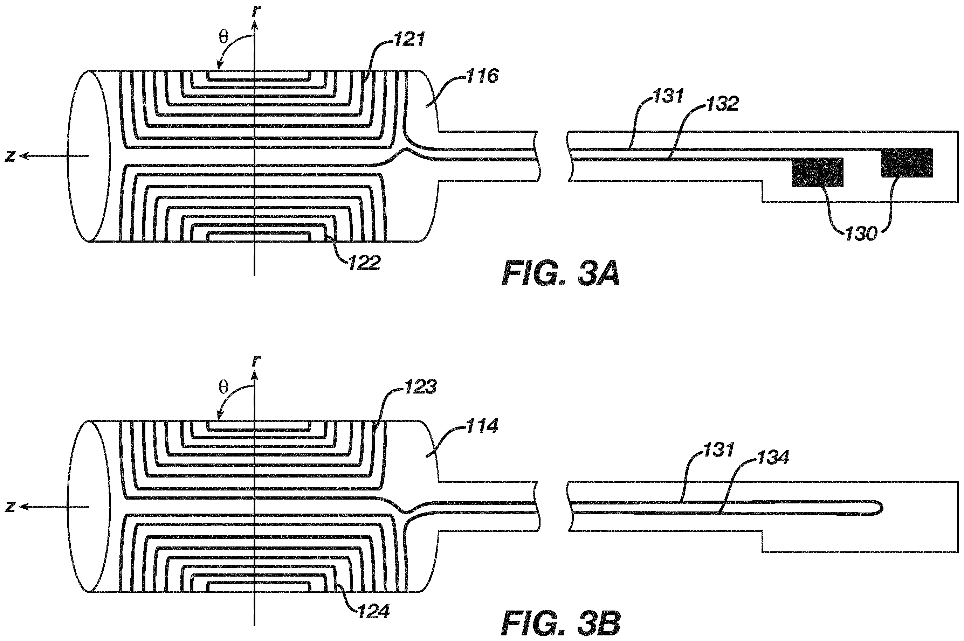

Figures 3A and 3B illustrate the first inductive sensor 120, associated traces 131-134, and contact pads 130. The first inductive sensor 120 as illustrated includes a first coil 121, a second coil 122, a third coil 123, and a fourth coil 124. The first and second coils 121, 122 are affixed to an insulating layer 116 as illustrated in Figure 3A. The third and fourth coils 123, 124 are affixed to a substrate 114 as illustrated in Figure 3B. The insulating layer 116 (Figure 3A) can be positioned over the third and fourth coils 123, 124 (Figure 3B) to form the first inductive sensor 120. The substrate 114 and insulating layer 116 are respectively formed in a cylindrical shape. The coils 121, 122, 123, 124 conform to the respective cylindrical shape. The second inductive sensor 140 and/or the third inductive sensor 160 can respectively include four coils arranged in a similar manner as the first, second, third, and fourth coils 121-124 of the first inductive sensor 120 as illustrated.

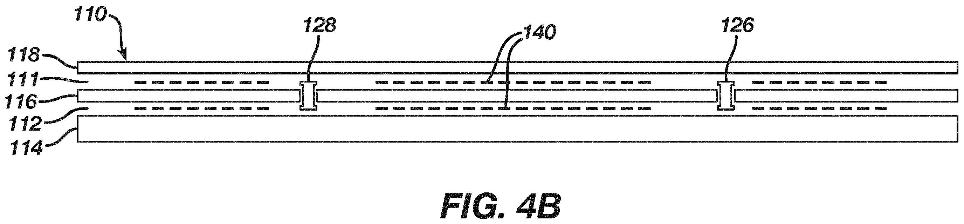

The substrate 114 can be affixed to the cylindrical surface 183 (Figures 2A and 2B). Configured as such, the first coil 121 and second coil 122 are adjacent neighbors; likewise, the third coil 123 and fourth coil are adjacent neighbors. The first and third coils 121, 123 are stacked neighbors, and the second and fourth coils 122, 124 are stacked neighbors. Each coil includes a central termination that is electrically connected to its stacked neighbor through a respective via 126, 128 (Figure 4B) through the insulating layer 116.

The first and second coils 121, 122 are positioned 180° apart from each other around the cylindrical surface 183 of the distal portion 108 when the circuit 110 is affixed to the catheter 100 as illustrated in Figures 1A through 2B. Likewise, the third and fourth coils 123, 124 are positioned 180° apart from each other around the cylindrical surface 183.

The third coil 123 can spiral oppositely from the first coil 121. The first coil 121 and third coil 123 can be positioned such that a majority of the first coil 121 overlaps a majority of the third coil 123. The fourth coil 124 can spiral oppositely from the second coil 122. The second coil 122 and the fourth coil 124 can be positioned such that a majority of the second coil 122 overlaps a majority of the fourth coil 124. The first coil 121 can spiral oppositely from the second coil 122. The third coil 123 can spiral oppositely from the fourth coil 124. The third coil 123 and the fourth coil 124 can be confined between two electrically insulative, substantially parallel, arcuate surfaces 114, 116.

The circuit 110 can further include conductive traces 131-134 to the coils 121-124. A first conductive trace 131 can be in immediate electrical contact with the first coil 121 and extend from the first coil 121 to the proximal shaft 106 (i.e. near a junction 105 between the distal portion 108 and proximal shaft 106 or further toward the proximal end 102 of the tubular body 103). A second conductive trace 132 can be in immediate electrical contact with the second coil 122 and extend from the second coil 122 to the proximal shaft 106. A third conductive trace 133 can be in immediate electrical contact with the third coil 123 and extend from the third coil 123 to the proximal shaft 106. The first and third conductive traces 131, 133 can be positioned such that a majority of the first conductive trace 131 overlaps a majority of the third conductive trace 133. A fourth conductive trace 134 can be in immediate electrical contact with the fourth coil 124 and can extend from the fourth coil 124 to the proximal shaft 106. The second and fourth conductive traces 132, 134 can be positioned such that a majority of the second conductive trace 132 overlaps a majority of the fourth conductive trace 134. The fourth conductive trace 134 can be electrically connected to the third conductive trace 133 near the proximal shaft 106. By extending the third and fourth conductive traces 133, 134 to run near the first and second conductive traces 131, 132, the traces can act similar to a twisted pair to reduce noise in the electrical signal from the first inductive sensor 120 compared to a configuration where the third and fourth traces 133 are foreshortened or otherwise routed in the circuit 110.

Geometry of the coils 121-124 and circuit 110 can be described in relation to a cylindrical coordinate system having a radial axis (r), z-axis (z), and azimuth (θ). The radial axis (r) is aligned with the coils 121-124 similarly to as illustrated in Figures 2A and 2B. The z-axis (z) is approximately coaxial with the polymer tube 182 illustrated in Figures 2A and 2B. Each of the coils 121-124 are curved so that they spiral generally at a constant radial distance from the z-axis. Each coil 121-124 spirals through an azimuth (θ) of less than 180° and preferably about 140° or more.

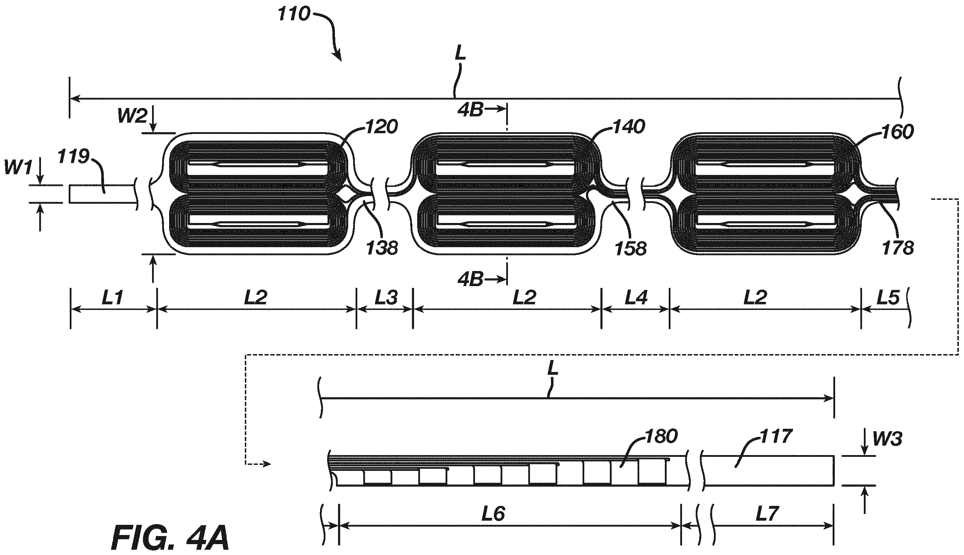

Figure 4A is an illustration of an example circuit 110 usable with the catheter 100. The circuit 110 is illustrated in a flat configuration and is flexible so that it can be wrapped around the distal portion 108 of the catheter body 103 as illustrated in Figures 1A through 3B. The circuit 110 can have a length (L) sufficient to be capable of positioning the sensors 120, 140, 160 on the distal portion 108 of the catheter body 103 and preferably position the contact pad arrangement 180 within the proximal shaft 106. In one example, the circuit 110 has a length (L) of about 20 cm.

The circuit 110 includes a distal segment 119 that lacks conductive traces. The distal segment can be shaped and otherwise configured to help anchor the circuit 110 to the cylindrical surface 183 of the distal portion 108. The distal segment 119 of the circuit 110 can have a width W1 measuring about 0.6 mm and a length L1 of about 25 mm. Each of the sensors 120, 140, 160 can respectively be positioned on portions of the circuit 110 each having a width W2 of about 3.2 mm and a length L2 of about 5 mm. The coil arrangements can occupy a majority of the area of those portions. The sensors 120, 140, 160 can be separated by intermediate segments 138, 158 having a width about equal to that of the distal segment 119 and a length L3, L4 of about 11 mm to about 13 mm. In one example, the length L4 between the third sensor 160 and the second sensor 140 can be about 13 mm and the length L3 between the second sensor 140 and first sensor 120 can be about 11 mm. The circuit 110 can include a proximal segment 178 between the third sensor 160 and a contact pad arrangement 180. The proximal segment 178 can have a length L5 measuring about 105 mm and a width measuring about the same as the distal segment 119 and intermediate segments 138, 158. The length L5 of the proximal segment 178 can be sufficient to position the contact pad arrangement 180 in the proximal shaft 106. The contact pad arrangement 180 can be positioned on a segment of the circuit 110 having a length L6 measuring about 9.25 mm and a width W3 measuring about 0.8 mm. The circuit 110 can include a proximal segment 117 with no conductive traces. The proximal segment 117 can have a length L7 measuring about 25 mm and a width W3 measuring about equal to the width of the segment of the circuit 110 including the contact pad arrangement 180 or can be narrower, about 0.6 mm.

Figure 4B is an illustration of the cross section of the circuit as indicated in Figure 4A. The circuit 110 can include an insulative substrate 114, a lower layer 112, an insulating mid layer 116, and an upper layer 111. The circuit 110 can further include an insulative top 118 layer above the upper layer 111. The insulative substrate 114 can be affixed to the cylindrical surface 183 of the distal portion 108 of the tubular body 103 of the catheter 100 to form the cross section illustrated in Figures 2A and 2B. The lower layer 112 can be above the insulative substrate 114 and can include the third coil 123, third conductive trace 133, fourth coil 124, and fourth conductive trace 134. The insulating mid layer 116 can be above the lower layer 112 and can include vias 126, 128 therethrough. The vias 126, 128 can facilitate immediate electrical contact between the central termination of the first coil 121 and the central termination of the third coil 123 and can facilitate immediate electrical contact between the central termination of the second coil 122 and the central termination of the fourth coil 124. The upper layer 111 above the insulating mid layer 116 can include the first coil 121, first conductive trace 131, second coil 122, and second conductive trace 132.

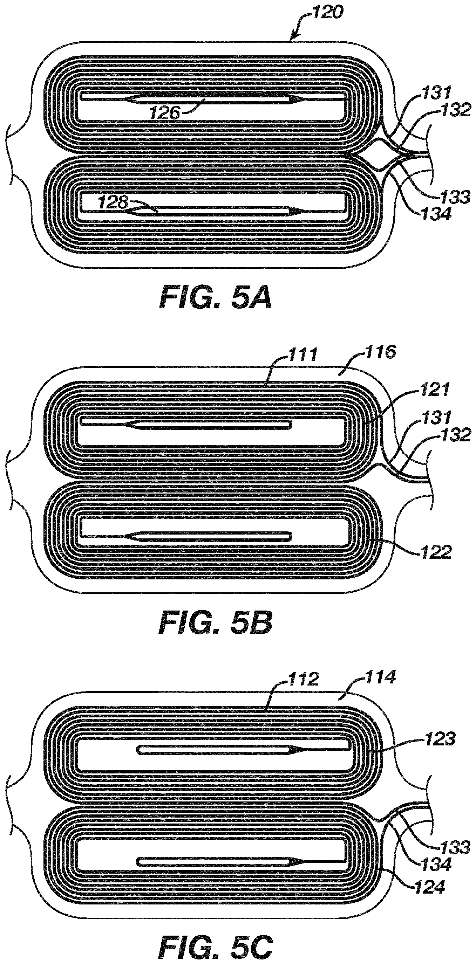

Figures 5A through 5C are illustrations of a first coil arrangement of the first inductive sensor 120 of the circuit 110. Figure 5A illustrates the first inductive sensor 120 assembled. Figure 5B illustrates the insulating separator 116 and first and second coils 121, 122 on the upper layer 111 of the circuit 110. Figure 5C illustrates the substrate 114 and the third and fourth coils 123, 124 on the lower layer 112. Coils are connected between layers 111, 112 by the vias 126, 128 at central terminations.

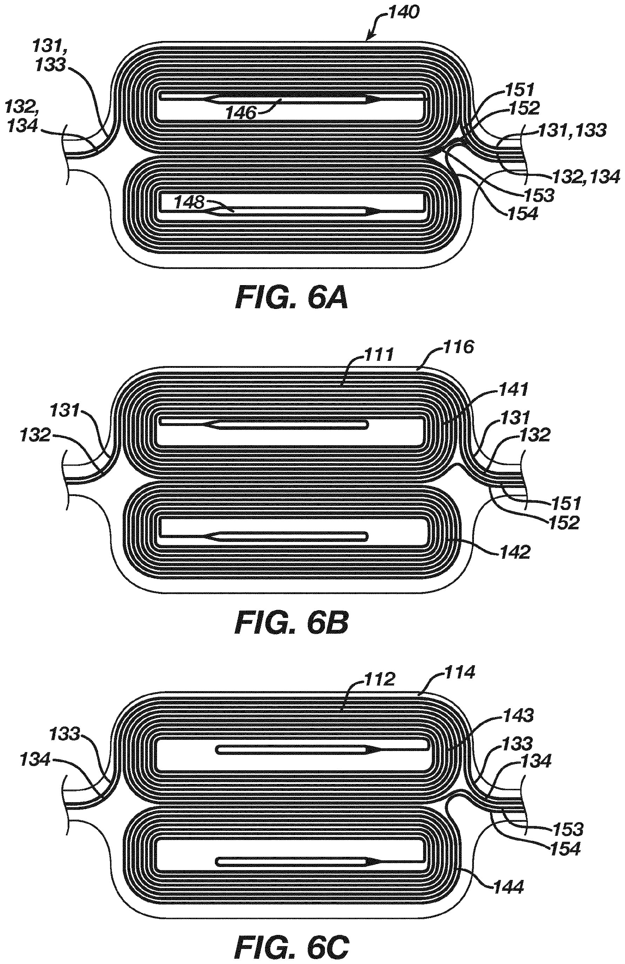

Figures 6A through 6C are illustrations of a second coil arrangement of the second inductive sensor 140 of the circuit 110. Figure 6A illustrates the second inductive sensor 140 assembled. Figure 6B illustrates the insulating separator 116 and fifth and sixth coils 141, 142 on the upper layer 111 of the circuit 110. Figure 6C illustrates the substrate 114 and the seventh and eighth coils 143, 144 on the lower layer 112. The traces 131-134 from the first inductive sensor 120 are routed around the second coil arrangement of the second inductive sensor 140. Coils are connected between layers 111, 112 by vias 146, 148 at central terminations.

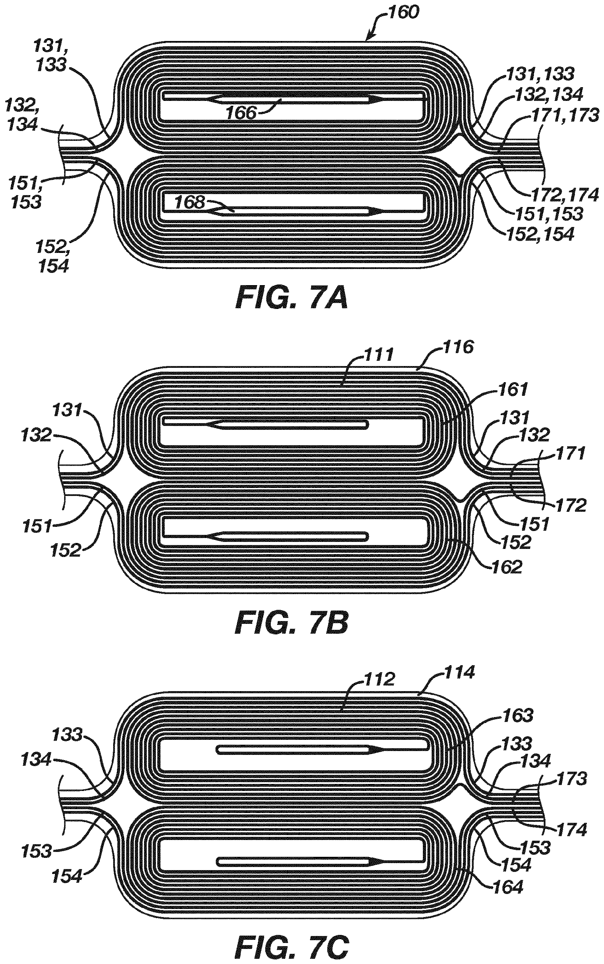

Figures 7A through 7C are illustrations of a third coil arrangement of the third inductive sensor 160 of the circuit 110. Figure 7A illustrates the third inductive sensor 160 assembled. Figure 7B illustrates the insulating separator 116 and ninth and tenth coils 161, 162 on the upper layer 111 of the circuit 110. Figure 7C illustrates the substrate 114 and eleventh and twelfth coils 163, 164 on the lower layer 112. The traces 131-134, 151-154 from the first inductive sensor 120 and the second inductive sensor 140 are routed around the third coil arrangement of the third inductive sensor 160. Coils are connected between layers 111, 112 by vias 166, 168 at central terminations.

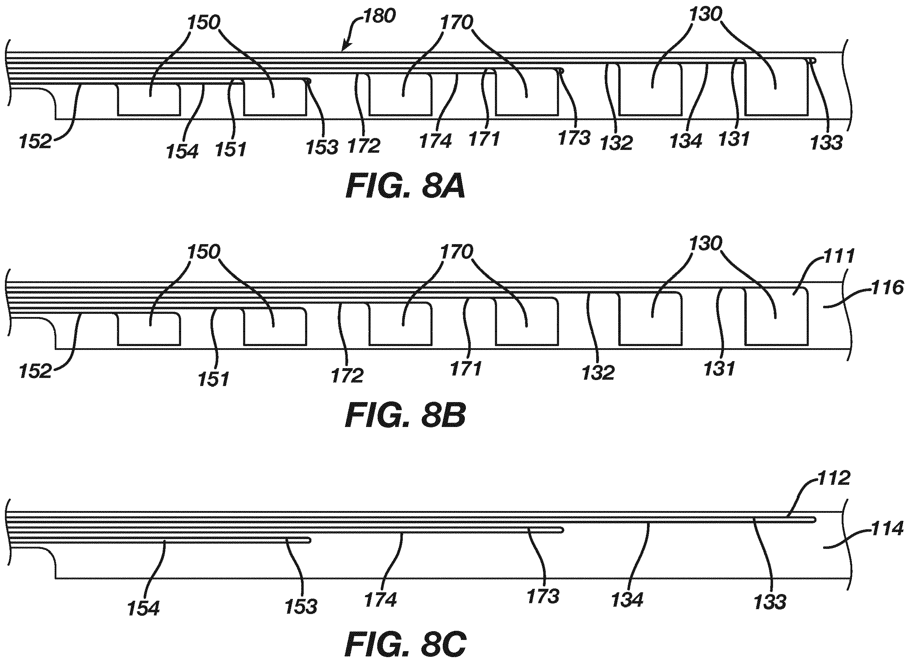

Figures 8A through 8C are illustrations of traces 131-134, 151-154, 171-174 and contact pads 130, 150, 170 of the circuit 110. Wires or other electrical conductors can be soldered to the contact pads 130, 150, 170 to make electrical connection to the coils 121-124, 141-144, 161-164. Such wires or conductors can extend through the proximal shaft 106 to a proximal end 102 of the tubular body 103 to make electrical signals from the sensors 120, 140, 160 accessible to equipment outside the patient.

Figure 8A illustrates the assembled contact pad arrangement 180. Figure 8B illustrates the insulating separator 116 and traces 131, 132, 151, 152, 171, 172 from the first coil 121, second coil 122, fifth coil 141, sixth coil 142, ninth coil 161, and tenth coil 162, which are the coils and traces on the upper layer 111 of the circuit 110. Contact pads 130, 150, 170 are also positioned in the upper layer 111, on the insulating separator 116. Figure 8C illustrates the substrate 114 and traces 133, 134, 153, 154, 173, 174 from the third coil 123, fourth coil 124, seventh coil 143, eighth coil 144, eleventh coil 163, and twelfth coil 164, which are the coils and traces on the lower layer 112 of the circuit 110. Although traces 133, 134, 153, 154, 173, 174 on the lower layer 112 can be foreshortened, elongating the traces so that they extend under corresponding traces 131, 132, 151, 152, 171, 172 on the upper layer 111 can improve electromagnetic compatibility. Similar to a twisted pair, the traces 133, 134, 153, 154, 173, 174 on the lower layer 112 can be shaped, positioned, and otherwise configured in relation to the corresponding traces 131, 132, 151, 152, 171, 172 on the upper layer 111 to reduce electromagnetic radiation from the traces 131-134, 151-154, 171-174, reduce crosstalk between neighboring traces, and/or improve rejection of external electromagnetic interference.

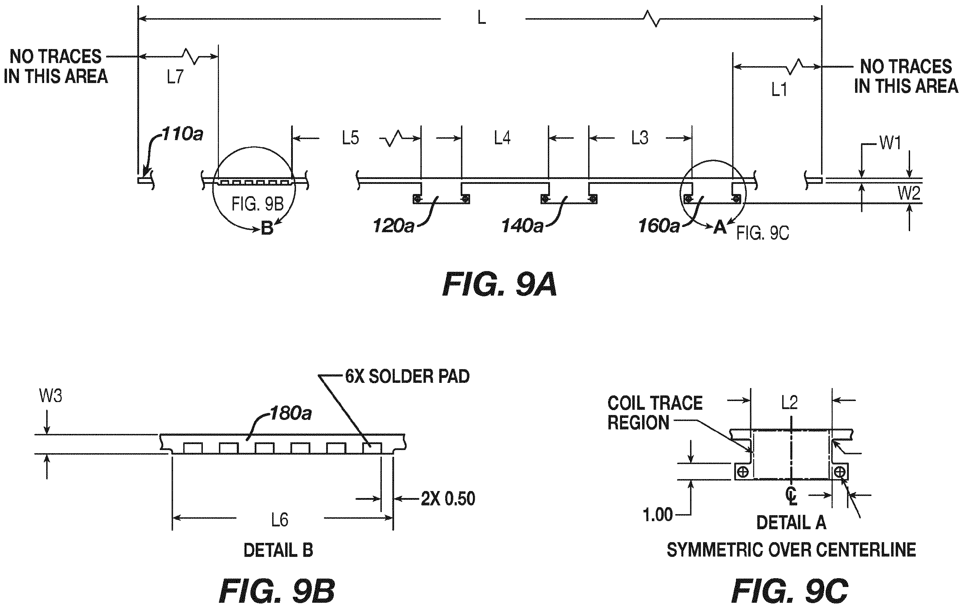

Figures 9A through 9C are illustrations of another example circuit 110a having sensors 120a, 140a, 160a and a contact pad arrangement 180a. The circuit 110a illustrated in Figures 9A through 9C can be usable in place of the circuit 110 illustrated in Figures 4A through 8C. Figure 9A is an overview illustration of the circuit 110a. Figures 9B and 9C are detailed views of portions of the circuit 110a as indicated in Figure 9A. The circuit 110a illustrated in Figures 9A through 9C can have dimensions L, L1-L7, W1-W3 similar to those of the circuit 110 illustrated in Figure 4A. The sensors 120a, 140a, 160a of the circuit 110a illustrated in Figures 9A through 9C can be configured similarly to the sensors 120, 140, 160 illustrated in Figures 1A through 8C. The circuit 110a illustrated in Figures 9A through 9C can be wrapped around the distal portion 108 of the catheter 100 to orient sensors 120a, 140a, 160a similarly to the orientation of corresponding sensors 120, 140, 160 of the circuit 110 illustrated in Figures 1A through 8C. Likewise, the circuit 110a can include coils, traces, and contact pads configured similarly to as described in relation to the circuit 110 illustrated in Figures 1A through 8C. Differences in geometry between the circuit 110a illustrated in Figures 9A through 9C and the circuit 110 illustrated in Figures 1A through 8C can be accommodated using techniques understood by a person skilled in the pertinent art according to the teachings herein. Likewise, other suitable circuits having various geometries can be realized by a person skilled in the pertinent art according to the teachings herein.

Figures 10A and 10B are illustrations of additional example catheters 100a, 100b. The distal portions 108a, 108b of the respective catheters 100a, 100b are in a generally circular shape similar to as illustrated and described in relation to the catheter 100 illustrated in Figure 1A. The catheters 100a, 100b illustrated in Figures 10A and 10B respectively include the circuit 110 including the sensors 120, 140, 160 illustrated in Figures 1A through 8C. The catheters 100a, 100b can alternatively include the circuit 110a illustrated in Figures 9A through 9C or a variation of the described circuits 110, 110a as understood by a person skilled in the pertinent art according to the teachings herein. The respective circular shape of the distal portion 108a, 108b of the respective catheters 100a, 100b illustrated in Figures 10A and 10B has a diameter D2 which can be similar to the diameter of the catheter 100 illustrated in Figure 1A, where diameter D2 is the circumference of the circular shape divided by pi.

The catheter 100b illustrated in Figure 10B includes mapping electrodes 188 positioned on the distal portion 108b. The mapping electrodes 188 can be configured similar to as described in U.S. Patent No. 6,987,995 incorporated by reference herein into this application as if set forth in full herein and which is attached in the Appendix to priority application U.S. 63/084,674 or can be configured in another suitable configuration as understood by a person skilled in the pertinent art. In some examples, the circular arrangement of the mapping electrodes 188 can permit measurement of the electrical activity so that ectopic beats between the electrodes can be identified. The size of the generally circular distal portion 108b can facilitate measurement of electrical activity within a circumference of a pulmonary vein or other tubular structure of, or near, the heart. The distal portion 108b can have a diameter D2 generally corresponding to that of a pulmonary vein, the coronary sinus, or other circular or tubular anatomical structure being diagnosed.

The mapping electrodes 188 can be made of a suitable conductive material, such as platinum or gold, preferably a combination of platinum and iridium. The mapping electrodes 188 include a series of ring electrodes mounted over the polymeric tube 182 of the distal portion 108 of the tubular body 103 of the catheter 100b. The mapping electrodes 188 can be mounted over the circuit 110 and over the sensors 120, 140, 160. The distal portion 108b can optionally include a non-conductive cover positioned over the circuit 110 and under the mapping electrodes 188. The mapping electrodes 188 can be affixed to the distal portion 108b with glue ,weld, crimp, or the like. Alternatively, the mapping electrodes 188 can be formed by coating the distal portion 108b with an electrically conducting material, like platinum, gold and/or iridium. The coating can be applied using sputtering, ion beam deposition or an equivalent technique. In some examples, each mapping electrode 188 is mounted by forming a hole in the polymeric tube 182, an electrode lead wire (not illustrated) is fed through the hole, and the mapping electrode 188 is welded in place over the lead wire and polymeric tube 182. The lead wires extend through the polymeric tube 182 and into the proximal shaft 106. The proximal end of each lead wire is electrically connected to a suitable connector (not shown), which is connected an appropriate monitor or other device for receiving and displaying the information received from the mapping electrodes 188. Alternatively, the mapping electrodes 188 can be formed by adding an upper layer on top of the flexible circuit 110, for instance by patterning conductors on top of the insulative top 118 layer (see Figure 4B).

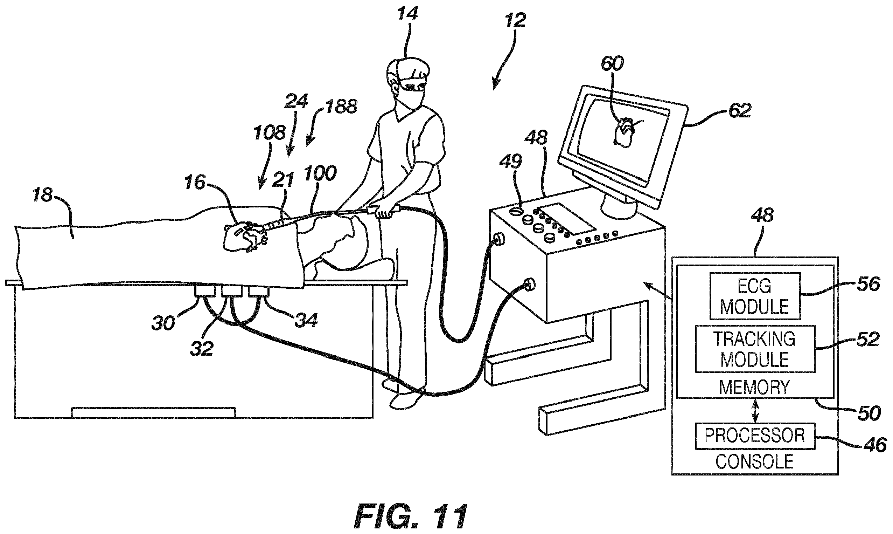

Figure 11 is an illustration of a medical treatment with an example system 12 incorporating an example catheter 100 which can be configured similarly to the example catheters 100, 100a-b illustrated herein, disclosed herein, or a variation thereof as understood by a person skilled in the pertinent art according to the teachings herein. The treatment is performed by a medical professional 14, and, by way of example, the procedure in the description hereinbelow is assumed to comprise an investigation of electropotentials a portion of a myocardium 16 of the heart of a human patient 18. However, example catheters 100, 100a-b are can be used in other medical treatment procedures as understood by a person skilled in the pertinent art.

In order to perform the investigation, the professional 14 inserts the catheter 100 into a sheath 21 that has been pre-positioned in a lumen of the patient. The sheath 21 is positioned so that the distal portion 108 of the catheter 100 enters the heart of the patient 18. The distal portion 108 include a position sensor 24 including three inductive sensors 120, 140, 160 as illustrated herein, disclosed herein, or a variation thereof as understood by a person skilled in the pertinent art according to the teachings herein. The position sensor 24 can enable tracking location and orientation of the distal portion 108 of the catheter 100. The distal portion 108 can also include mapping electrodes 188 as illustrated herein, disclosed herein, or a variation thereof as understood by a person skilled in the pertinent art according to the teachings herein. The mapping electrodes 188 can be used to acquire electropotentials of the myocardium 16.

The position sensor 24 includes inductive sensors 120, 140, 160 which respectively include a plurality of coils 121-124, 141-144, 161-164. While the description herein describes using the coils for sensing magnetic fields, the coils may also be used to produce magnetic fields.

The system 12 can include a console 48 having a system processor 46. The console 48 can include controls 49 which can be usable by the professional 14 to communicate with the processor 46. The software for the processor 46 can be downloaded to the processor in electronic form, over a network, for example. Alternatively, or additionally, the software can be provided on non-transitory tangible media, such as optical, magnetic, or electronic storage media. Tracking (e.g. position and orientation) of distal portion 108 of the catheter 100 can be displayed on a three-dimensional representation 60 of the heart of patient 18 that is displayed on a screen 62.

In order to operate the system 12, the processor 46 communicates with a memory 50, which has a number of modules used by the processor 46 to operate the system 12. Thus, the memory 50 can include an electrocardiograph (ECG) module 56 which acquires and analyzes signals from the mapping electrodes 188. The memory 50 can also include a tracking module 52, which receives signals from the position sensor 24, and which analyzes the signals in order to generate the location and orientation of distal portion 108. An ECG module 56 and the tracking module 52 can include hardware and/or software components. The memory 50 can include other software modules, such as a force module for measuring the force on the distal portion 108, and/or an irrigation module allowing the processor 46 to control irrigation provided for the distal portion 108. For simplicity, such other modules are not illustrated in Figure 11.

In addition to receiving and analyzing signals from the position sensor 24, the tracking module 52 can also control radiators 30 32, 34. The radiators can be positioned in proximity to myocardium 16 and can be configured to radiate alternating magnetic fields into a region in proximity to the myocardium 16. The position sensor 24 can be configured to produce electrical signals which can be transmitted to the console 48 to be interpreted by the tracking module 52 to determine a three-dimensional position and orientation of the distal portion 108 of the catheter 100. Each of the inductive sensors 120, 140, 160 can be configured to generate the electrical signals of the position sensor 24 in response to the radiated magnetic fields traversing coils 121-124, 141-144, 161-164 of the inductive sensors 120, 140, 160, thereby enabling the console 48 to track the distal portion 108. The Carto^{®} system produced by Biosense Webster uses such a magnetic tracking system.

In many known magnetic tracking systems, three inductive sensors of a position sensor are aligned orthogonal to each other, i.e. coils of each of the respective inductive sensors are each aligned along a respective coil axis and each coil axis of an inductive sensor is orthogonal to the coil axis of the other two inductive sensors. In many known magnetic systems, coils are either planar (spiraling in a flat plane at an expanding radius from a central terminal) or cylindrically helical (spiraling along a length of a cylindrical shape at a constant radius from a central axis of the cylindrical shape). As presented herein, the sensors 120, 140, 160 of example catheters 100, 100a-b need not be orthogonal to each other. The coils 121-124, 141-144, 161-164 of the sensors 120, 140, 160 need not be planar nor cylindrically helical. The tracking module 52 can therefore be configured to determine a three-dimensional position of the distal portion 108 of the catheter 100 based on electrical signals from non-orthogonal sensors 120, 140, 160 and/or coils that are neither planar nor cylindrically helical.

The descriptions contained herein are examples of embodiments of the invention and are not intended in any way to limit the scope of the invention. As described herein, the invention contemplates many variations and modifications of the catheter 100, 100a-b, circuit 110, 100a, and methods for manufacturing and using the same. Additional modifications that are apparent to those having skill in the art to which this invention pertains and are intended to be within the scope of the claims which follow.

### ASPECT OF THE INVENTION

1. A method for intracardiac diagnostics, comprising:
   manipulating a proximal shaft of a catheter to position a distal portion of the catheter within a heart;
   receiving position signals from inductive coil arrangements affixed to a cylindrical surface of the distal portion, the coil arrangements shaped to have an arcuate cross-section as a result of being affixed to the cylindrical surface; and
   determining position and orientation of the distal portion based at least in part on the position signals.

## Claims

1. A catheter comprising:
a tubular body comprising a proximal shaft, a distal portion, and a delivery configuration in which the distal portion and proximal shaft are aligned along a longitudinal axis,
the proximal shaft being configured to be manipulated to deliver the distal portion through vasculature,
the distal portion comprising a cylindrical surface comprising a curvature around the longitudinal axis when the tubular body is in the delivery configuration; and
a circuit comprising a first inductive sensor, a second inductive sensor, and a third inductive sensor that are collectively configured to function as a three-axis sensor,
the first inductive sensor comprising first inductive coils spiraling substantially parallel to the cylindrical surface such that the first inductive coils conform to the cylindrical surface,
the second inductive sensor comprising second inductive coils spiraling substantially parallel to the cylindrical surface such that the second inductive coils conform to the cylindrical surface, and
the third inductive sensor comprising third inductive coils spiraling substantially parallel to the cylindrical surface such that the third inductive coils conform to the cylindrical surface.

2. The catheter of claim 1, the tubular body comprising a deployed configuration in which the distal portion comprises a generally circular shape generally orthogonal to the longitudinal axis,
the tubular body being movable from the delivery configuration to the deployed configuration via manipulation of the proximal shaft, and
the first inductive sensor, second inductive sensor, and third inductive sensor being each spaced approximately equidistant from each other around the generally circular shape.

3. The catheter of claim 2, the generally circular shape comprising a circumference measuring approximately 50 millimeters.

4. The catheter of claim 2, further comprising:
a support member extending through a tubular lumen of the tubular body within the distal portion,
the tubular body comprising a flexible polymeric material.

5. The catheter of claim 4, further comprising:
a contraction wire extending through the tubular lumen of the tubular body within the distal portion, the contraction wire movable to modify a shape of the distal portion to the generally circular shape,
the support member comprising a memory shape material and a predetermined shape approximate to the generally circular shape.

6. The catheter of claim 1, the first inductive sensor, the second inductive sensor, and the third inductive sensor each lacking any inductive coil circumscribing the cylindrical surface.

7. The catheter of claim 1, the first inductive coils comprising:
a first coil positioned on a first side of the cylindrical surface and comprising a central termination;
a second coil positioned on a second side of the cylindrical surface, about 180° around the cylindrical surface from the first side and comprising a central termination;
a third coil spiraling oppositely from the first coil, positioned on the first side of the cylindrical surface, positioned such that a majority of the first coil overlaps a majority of the third coil, and comprising a central termination in immediate electrical contact with the central terminal of the first coil; and
a fourth coil spiraling oppositely from the second coil, positioned on the second side of the cylindrical surface, positioned such that a majority of the second coil overlaps a majority of the fourth coil, and comprising a central termination in immediate electrical contact with the central terminal of the second coil.

8. The catheter of claim 7,
the first coil spiraling oppositely from the second coil, and
the third coil spiraling oppositely from the fourth coil.

9. The catheter of claim 7, the third coil and the fourth coil being confined between two electrically insulative, substantially parallel, arcuate surfaces.

10. The catheter of claim 7, further comprising:
a first conductive trace in immediate electrical contact with the first coil and extending from the first coil to the proximal shaft;
a second conductive trace in immediate electrical contact with the second coil and extending from the second coil to the proximal shaft;
a third conductive trace in immediate electrical contact with the third coil, extending from the third coil to the proximal shaft, and positioned such that a majority of the first conductive trace overlaps a majority of the third conductive trace; and
a fourth conductive trace in immediate electrical contact with the fourth coil, extending from the fourth coil to the proximal shaft, positioned such that a majority of the second conductive trace overlaps a majority of the fourth conductive trace, and electrically connected to the third conductive trace approximate the proximal shaft.

11. The catheter of claim 10, the circuit comprising:
an insulative substrate affixed to the cylindrical surface;
a lower layer above the insulative substrate and comprising the third coil, third conductive trace, fourth coil, and fourth conductive trace;
an insulating mid layer above the lower layer comprising vias therethrough, the vias facilitating immediate electrical contact between a central termination of the first coil and a central termination of the third coil and facilitating immediate electrical contact between a central termination of the second coil and a central termination of the fourth coil;
an upper layer above the insulating mid layer comprising the first coil, first conductive trace, second coil, and second conductive trace; and
an insulative top layer above the upper layer.

12. The catheter of claim 11, further comprising:
a first contact pad positioned in the upper layer approximate the proximal shaft;
a first wire soldered to the first contact pad and extending through the proximal shaft to a proximal end of the tubular body;
a second contact pad positioned in the upper layer approximate the proximal shaft; and
a second wire soldered to the second contact pad and extending through the proximal shaft to the proximal end of the tubular body.

13. The catheter of claim 1, the first inductive coils, the second inductive coils, and the third inductive coils each respectively spiraling around a respective coil axis such that each respective coil axis is approximately orthogonal to the cylindrical surface.

14. The catheter of claim 13, each of the first inductive coils comprising a height measured in a direction of the respective coil axis of the first inductive coils and a width measured orthogonal to the respective coil axis, the width measuring at least ten times greater than the height.

15. A method comprising:
fabricating a multi-layer flexible circuit comprising a first coil arrangement, a second coil arrangement, and a third coil arrangement,
such that the first, second, and third coil arrangement are linearly arranged to define a longitudinal axis of the multi-layer flexible circuit,
such that each of the first, second, and third coil arrangement each comprise four coils,
such that the four coils each comprise a central termination and arranged such that each of the four coils is next to an adjacently stacked coil and an adjacent coplanar coil, and
such that the central termination of each of the four coils is in immediate electrical contact with its adjacently stacked coil; and
affixing the multi-layer flexible circuit to a cylindrical surface of a tubular catheter body such that the longitudinal axis of the multi-layer flexible circuit is aligned lengthwise with the tubular catheter body and such that, through each of the first, second, and third coil arrangements, the flexible circuit comprises a respective arcuate cross section orthogonal to the longitudinal axis.

16. The method of claim 15, further comprising:
affixing the multi-layer flexible circuit to the cylindrical surface such that each of the four coils is centered about 180° around a circumference of the tubular body from its adjacent coplanar coil.

17. The method of claim 15, further comprising:
forming a distal portion of the tubular catheter body in a circular shape; and
affixing the multi-layer flexible circuit to the cylindrical surface such that each of the first, second, and third coil arrangements are spaced approximately equidistant from each other around the generally circular shape when the distal portion is in the circular shape.

18. The method of claim 15, further comprising:
affixing the multi-layer flexible circuit to the cylindrical surface such that the first coil arrangement, the second coil arrangement, and the third coil arrangement each lack any inductive coil circumscribing the cylindrical surface.

19. The method of claim 15, further comprising:
affixing the multi-layer flexible circuit to the cylindrical surface such that the first coil arrangement comprises first inductive coils spiraling substantially parallel to the cylindrical surface so that the first inductive coils conform to the cylindrical surface, such that the second coil arrangement comprises second inductive coils spiraling substantially parallel to the cylindrical surface so that the second inductive coils conform to the cylindrical surface, and such that the third coil arrangement comprises third inductive coils spiraling substantially parallel to the cylindrical surface so that the third inductive coils conform to the cylindrical surface.
